# EUROPEAN PATENT APPLICATION

(11) **EP 4 478 375 A1**
(43) Date of publication of application: **18.12.2024**
(21) Application number: 23179268.0
(22) Date of filing: 14.06.2023
(51) Int. Cl.: G16H 40/63, A61B 5/00

(54) **METHOD FOR DETERMINING TREATMENT DATA RELATING TO ENABLED TREATMENT CONDITION OF PERSONAL CARE DEVICE**

(71) Applicant: Koninklijke Philips N.V., 5656 AG Eindhoven (NL)
(72) Inventor: FLINSENBERG, Ingrid Christina Maria, Eindhoven (NL); VAN ZUTPHEN, Martijn, Eindhoven (NL)
(74) Representative: Philips Intellectual Property & Standards

(57) **Abstract**

The invention provides a computer-implemented method (101, 301) for determining treatment data relating to treatment by means of a personal care device (1, 205). The method involves receiving (103, 303) first sensor data (SD₁) generated by a first sensor (13, 215) and relating to a treatment parameter at a first moment of time (t₁), and receiving (103, 303) second sensor data (SD₂) generated by a second sensor (19, 229) and relating to a treatment condition of the personal care device with respect a body part subject of the treatment. If, based on the second sensor data, the personal care device is determined to be in an enabled treatment condition at said first moment of time wherein the treatment is enabled, the treatment data are determined (309) based on the first sensor data relating to the treatment parameter at said first moment of time. The method further involves, by comparing (105, 305) the second sensor data with first predetermined sensor data (PSD₁), determining (105, 307) whether the second sensor is in a disabled condition at said first moment of time (t₁). If the second sensor is determined to be in the disabled condition at said first moment of time, a predefined data usage rule is used to determine (109, 111, 113; 313) whether to base the treatment data on the first sensor data (SD₁) relating to the treatment parameter at said first moment of time.

## Description

### FIELD OF THE INVENTION

The invention relates to a computer-implemented method for determining treatment data relating to treatment of a body part of a subject by means of a personal care device, the method comprising receiving first sensor data generated by a first sensor associated with the personal care device and relating to a treatment parameter of the treatment at a first moment of time; receiving second sensor data generated by a second sensor associated with the personal care device and relating to a treatment condition of the personal care device with respect to the body part indicating whether the treatment by the personal care device is enabled at said first moment of time; processing the second sensor data to determine whether, at said first moment of time, the personal care device is in an enabled treatment condition with respect to the body part wherein the treatment is enabled; and, if the personal care device is determined to be in the enabled treatment condition at said first moment of time, determining the treatment data based on the first sensor data relating to the treatment parameter at said first moment of time.

The invention further relates to a personal care device configured to provide a personal care treatment to a body part of a subject, comprising a first sensor configured to generate first sensor data relating to a treatment parameter of the personal care treatment at a first moment of time; a second sensor configured to generate second sensor data relating to a treatment condition of the personal care device with respect to the body part indicating whether the personal care treatment by the personal care device is enabled at said first moment of time; and a processor configured to receive the first sensor data and the second sensor data; process the second sensor data to determine whether, at said first moment of time, the personal care device is in an enabled treatment condition with respect to the body part enabling the personal care treatment; and, if the personal care device is determined to be in the enabled treatment condition at said first moment of time, determine treatment data relating to the personal care treatment based on the first sensor data relating to the treatment parameter at said first moment of time.

The invention further relates to a personal care system comprising a personal care device configured to provide a personal care treatment to a body part of a subject; a first sensor associated with the personal care device configured to generate first sensor data relating to a treatment parameter of the personal care treatment at a first moment of time; a second sensor associated with the personal care device configured to generate second sensor data relating to a treatment condition of the personal care device with respect to the body part indicating whether the personal care treatment by the personal care device is enabled at said first moment of time; and a processor configured to receive the first sensor data and the second sensor data; process the second sensor data to determine whether, at said first moment of time, the personal care device is in an enabled treatment condition with respect to the body part enabling the personal care treatment; and, if the personal care device is determined to be in the enabled treatment condition at said first moment of time, determine treatment data relating to the personal care treatment based on the first sensor data relating to the treatment parameter at said first moment of time.

### BACKGROUND OF THE INVENTION

Personal care devices configured to provide a personal care treatment to a body part of a subject are widely known. Examples are electric shavers to shave beard hairs on the face of a human, hair-trimming devices to cut beard hairs on the face of a human or hairs on human body parts other than the face, intense pulsed light (IPL) devices to remove hairs on the legs or other body parts of a human, facial-cleansing brushing devices to clean the face of a human by means of a rotating and/or vibrating brush, and skin rejuvenation devices to rejuvenate the skin of body parts of a human by means of electromagnetic or radiofrequency energy.

A personal care device may have a first sensor configured to generate first sensor data relating to a treatment parameter of the personal care treatment. Treatment data may be determined based on one or more treatment parameters of the personal care treatment measured by the first sensor. Such treatment data may be useful, for example to inform the user of the personal care device about the treatment data (such as treatment progress or treatment results) or to adjust one or more operational parameters of the personal care device as a function of the treatment data. Examples of useful treatment data are a momentary location of the personal care device on the body part, a sequence of locations of the personal care device on the body part during a treatment session, and a dwell time of the personal care device for each of a plurality of segments of the body part during a treatment session.

For the determination of such treatment data, it may further be useful to disregard first sensor data generated by the first sensor during periods of a treatment session wherein the personal care device is disabled to perform the personal care treatment, e.g., because the user has not yet started the personal care treatment or has paused the personal care treatment by moving the personal care device away from the body part. For this purpose, the personal care device may have a second sensor configured to generate second sensor data relating to a treatment condition of the personal care device with respect to the body part indicating whether the personal care treatment by the personal care device is enabled. An example of such a treatment condition is physical contact of the personal care device with the body part, which can be detected by means of, for example, a skin proximity sensor or a skin pressure sensor. If, based on the second sensor data received for a certain period during a treatment session, the personal care device is determined to be in a condition which does not enable the personal care treatment (e.g., no contact with the body part), the first sensor data received during said period may be disregarded when determining the treatment data relating to the personal care treatment. This will increase the accuracy of the treatment data.

A problem associated with a personal care device as described here before is that the user may inadvertently disable the second sensor from properly measuring the treatment condition of the personal care device with respect to the body part indicating whether the personal care treatment by the personal care device is enabled. The user may disable the second sensor, for example, by improperly holding the personal care device or, in case of an optical sensor, by blocking an optical window of the second sensor by means of a finger. In such a disabled condition, the second sensor may generate second sensor data indicative of a disabled treatment condition of the personal care device with respect to the body part wherein the personal care treatment is not enabled, or second sensor data similar to second sensor data indicative of a disabled treatment condition of the personal care device. As a result, the first sensor data, generated by the first sensor during the period wherein the second sensor is in the disabled condition, may be disregarded when determining the treatment data, although the personal care treatment might be enabled during said period. As a result, the treatment data may become inaccurate or at least incomplete.

### SUMMARY OF THE INVENTION

It is an object of the present invention to provide a computer-implemented method, a personal care device and a personal care system, each having the features as mentioned in the section "field of the invention" here before, by means of which the problem associated with the known personal care device as described here before may at least be alleviated, so that the treatment data determined according to the method or by means of the processor of the personal care device or the personal case system are more accurate, or at least more complete, and better represent the actual personal care treatment carried out by the personal care device.

According to a first aspect of the invention, to achieve the above-mentioned object a computer-implemented method having the features as mentioned in the section "field of the invention" here before is characterized in that the method further comprises comparing the second sensor data with first predetermined sensor data characteristic of a disabled condition of the second sensor wherein the second sensor is disabled from measuring the treatment condition of the personal care device with respect to the body part; determining, based on said comparing, whether the second sensor is in the disabled condition at said first moment of time; and, if the second sensor is determined to be in the disabled condition at said first moment of time, determining, based on a predefined data usage rule, whether to base the treatment data on the first sensor data relating to the treatment parameter at said first moment of time.

According to a second aspect and a third aspect of the invention, to achieve the above-mentioned object a personal care device and a personal care system, each having the features as mentioned in the section "field of the invention" here before, are each characterized in that the processor is further configured to compare the second sensor data with first predetermined sensor data characteristic of a disabled condition of the second sensor wherein the second sensor is disabled from measuring the treatment condition of the personal care device with respect to the body part; determine, based on said comparing, whether the second sensor is in the disabled condition at said first moment of time; and, if the second sensor is determined to be in the disabled condition at said first moment of time, determine, based on a predefined data usage rule, whether to base the treatment data on the first sensor data relating to the treatment parameter at said first moment of time.

Accordingly, the processor of the personal care device according to the second aspect of the invention and the processor of the personal care system according to the third aspect of the invention are each configured (e.g., programmed) to perform the computer-implemented method according to the first aspect of the invention. While the personal care device according to the second aspect of the invention comprises the first sensor, the second sensor and the processor, in the personal care system according to the third aspect of the invention the first and second sensors associated with the personal care device and the processor may each be part of the personal care device or part of another device separate from the personal care device. In the personal care system, e.g., the personal care device may comprise the first and second sensors and the processor may, e.g., be part of a smart phone or another electronic computing or processing device in data communication with the personal care device. Furthermore, in the personal care system the first sensor and/or the second sensor may be part of a measuring device separate from the personal care device and in data communication with the processor.

According to the method of the invention, the second sensor data generated by the second sensor are compared with first predetermined sensor data which are characteristic of a disabled condition of the second sensor wherein the second sensor is disabled from measuring the treatment condition of the personal care device with respect to the body part. The first predetermined sensor data may, e.g., be stored in a memory of a processor. When the second sensor is, e.g., an optical sensor, the first predetermined sensor data may be characteristic of a condition wherein the user blocks an optical window of the second sensor by a finger. By comparing the second sensor data generated by the second sensor at said first moment of time with said first predetermined sensor data, it is possible to determine whether the second sensor is (or was) in the disabled condition at said first moment of time.

Furthermore, according to the method of the invention a predefined data usage rule is applied if the second sensor is determined to be in the disabled condition at said first moment of time. Based on said predefined data usage rule, it is determined whether the treatment data are to be based also on the first sensor data generated by the first sensor at said first moment of time. Thus, based on the predefined data usage rule, the treatment data may be determined based on both first sensor data generated by the first sensor during moments of time for which the personal care device is determined, by means of the second sensor, to be in the enabled treatment condition and first sensor data generated by the first sensor during moments of time for which the second sensor is determined to be in the disabled condition. The data usage rule may be predefined based on, e.g., particular properties of the second sensor and insights or assumptions about the way of handling of the personal care device by the user during a treatment session. Examples of the predefined data usage rule will be described here after with respect to embodiments of the invention. In any case, the predefined data usage rule allows the treatment data to be based also on first sensor data generated by the first sensor during periods wherein the second sensor is in the disabled condition, so that the treatment data determined by means of the method are at least more complete, may be more accurate, and may better represent the actual personal care treatment carried out by the personal care device during a treatment session.

In an embodiment of the computer-implemented method according to the invention, the method comprises receiving a plurality of the first sensor data and a plurality of the second sensor data relating to, respectively, the treatment parameter and the treatment condition of the personal care device at a plurality of successive moments of time during a treatment session; determining for each moment of time of said plurality of successive moments of time, based on said comparing, whether the second sensor is in the disabled condition; and, after receiving said plurality of the first sensor data and said plurality of the second sensor data, determining a portion of said plurality of successive moments of time for which the second sensor is determined to be in the disabled condition; wherein the predefined data usage rule causes the method to base the treatment data on the first sensor data received for each moment of time of said plurality of successive moments of time if said determined portion is above a predefined threshold, and on only the first sensor data received for the moments of time of said plurality of successive moments of time for which the second sensor is determined not to be in the disabled condition if said determined portion is below said predefined threshold.

In embodiments of the personal care device and the personal care system according to the invention, the processor is further configured to receive a plurality of the first sensor data and a plurality of the second sensor data relating to, respectively, the treatment parameter and the treatment condition of the personal care device at a plurality of successive moments of time during a treatment session; to determine for each moment of time of said plurality of successive moments of time, based on said comparing, whether the second sensor is in the disabled condition; and, after receiving said plurality of the first sensor data and said plurality of the second sensor data, to determine a portion of said plurality of successive moments of time for which the second sensor is determined to be in the disabled condition; wherein the predefined data usage rule causes the processor to base the treatment data on the first sensor data received for each moment of time of said plurality of successive moments of time if said determined portion is above a predefined threshold; and on only the first sensor data received for the moments of time of said plurality of successive moments of time for which the second sensor is determined not to be in the disabled condition if said determined portion is below said predefined threshold.

These embodiments of the computer-implemented method, the personal care device and the personal care system according to the invention are particularly suitable when the second sensor is of a type for which the second sensor data generated in the disabled condition of the second sensor are identical or similar to the second sensor data generated, in the enabled condition of the second sensor, for the disabled treatment condition of the personal care device with respect to the body part. With this type of second sensor, based on the second sensor data the disabled condition of the second sensor cannot be distinguished with sufficient accuracy from the disabled treatment condition of the personal care device measured in the enabled condition of the second sensor. According to these embodiments it is proposed to first receive the first sensor data and the second sensor data for a plurality of successive moments of time, e.g., for an entire treatment session of the personal care device, and thereafter to determine, based on the received second sensor data, a portion of said plurality of successive moments of time for which the second sensor is (or was) in the disabled condition. In these embodiments, according to the predefined data usage rule, if said determined portion is above a predefined threshold the treatment data are determined based on the first sensor data received for each moment of time of said plurality of successive moments of time. If said determined portion is below said predefined threshold the treatment data are determined based on only the first sensor data received for the moments of time of said plurality of successive moments of time for which the second sensor is determined not to be in the disabled condition, i.e., for which, based on the second sensor data, the personal care device is determined to be in the enabled treatment condition. In these embodiments the predefined data usage rule is based on the assumption that, if a user disables the second sensor during a relatively long period of the treatment session, the personal care device will most likely be in the enabled treatment condition for a larger portion of said period, so that it makes most sense to base the treatment data also on the first sensor data received during said period.

In a further embodiment of the computer-implemented method according to the invention, the method comprises comparing the second sensor data with second and third predetermined sensor data characteristic of an enabled condition of the second sensor wherein the second sensor is enabled to measure the treatment condition of the personal care device with respect to the body part, wherein the second predetermined sensor data are characteristic of the enabled treatment condition of the personal care device, and wherein the third predetermined sensor data are different from the first predetermined sensor data and characteristic of a disabled treatment condition of the personal care device with respect to the body part wherein the treatment is not enabled; determining, based on said comparing of the second sensor data with said second and third predetermined sensor data, whether the personal care device is in the disabled treatment condition at said first moment of time; and, if the personal care device is determined to be in the disabled treatment condition at said first moment of time, disregarding the first sensor data relating to the treatment parameter at said first moment of time when determining the treatment data; wherein the predefined data usage rule causes the method to base the treatment data on the first sensor data relating to the treatment parameter at said first moment of time if the second sensor is determined to be in the disabled condition at said first moment of time.

In a further embodiments of the personal care device and the personal care system according to the invention, the processor is further configured to compare the second sensor data with second and third predetermined sensor data characteristic of an enabled condition of the second sensor wherein the second sensor is enabled to measure the treatment condition of the personal care device with respect to the body part, wherein the second predetermined sensor data are characteristic of the enabled treatment condition of the personal care device, and wherein the third predetermined sensor data are different from the first predetermined sensor data and characteristic of a disabled treatment condition of the personal care device with respect to the body part wherein the treatment is not enabled; to determine, based on said comparing of the second sensor data with said second and third predetermined sensor data, whether the personal care device is in the disabled treatment condition at said first moment of time; and, if the personal care device is determined to be in the disabled treatment condition at said first moment of time, disregard the first sensor data relating to the treatment parameter at said first moment of time when determining the treatment data; wherein the predefined data usage rule causes the processor to base the treatment data on the first sensor data relating to the treatment parameter at said first moment of time if the second sensor is determined to be in the disabled condition at said first moment of time.

These embodiments of the computer-implemented method, the personal care device and the personal care system according to the invention are particularly suitable when the second sensor is of a type for which the second sensor data generated in the disabled condition of the second sensor are different from the second sensor data generated, in the enabled condition of the second sensor, for the disabled treatment condition of the personal care device. Thus, with this type of second sensor, based on the second sensor data the disabled condition of the second sensor can be distinguished with sufficient accuracy from the disabled treatment condition of the personal care device measured in the enabled condition of the second sensor. In these embodiments the first sensor data received for those moments of time for which the personal care device is determined to be in a disabled treatment condition are disregard when determining the treatment data. In these embodiments, according to the predefined data usage rule, the treatment data are also based on the first sensor data received for those moments of time for which the second sensor is determined to be in the disabled condition. The predefined data usage rule is based on the assumption that, if a user disables the second sensor during a treatment session, it is most likely that the personal care device will be in the enabled treatment condition with respect to the body part, so that it makes most sense to base the treatment data also on the first sensor data received when the second sensor is in the disabled condition. In these embodiments the treatment data may be determined based on the first sensor data in real-time during a treatment session.

The method according to the invention is particularly suitable for embodiments of a personal care device and a personal care system according to the invention wherein the enabled treatment condition of the personal care device involves a condition of the personal care device in contact with the body part, wherein the second sensor comprises a proximity sensor arranged in the personal care device such that, in the enabled treatment condition, the proximity sensor is at a predefined distance from a surface of the body part, and wherein the proximity sensor is configured to detect the presence of said surface at said predefined distance from the proximity sensor. In these embodiments, the proximity sensor is used to detect a condition wherein the surface of the body part to be treated is present at said predefined distance from the proximity sensor, which corresponds to the enabled treatment condition of the personal care device in contact with the surface of the body part. Because the proximity sensor is arranged at a distance from the surface of the body part, the proximity sensor can inadvertently be disabled by the user. When the proximity sensor is an optical sensor, the user can, e.g., inadvertently place a finger on the window of the optical sensor. For most types of a proximity sensor it is possible to distinguish, based on the second sensor data, the disabled condition of the proximity sensor from the disabled treatment condition of the personal care device measured in the enabled condition of the proximity sensor.

The method according to the invention is also particularly suitable for embodiments of a personal care device and a personal care system according to the invention wherein the personal care device comprises a main housing configured to be handheld by a user and a personal care treatment unit coupled to the main housing by means of a coupling structure, wherein the enabled treatment condition of the personal care device involves a condition of the personal care treatment unit in contact with the body part, wherein the second sensor comprises a pressure or force sensor configured to detect a pressure or force with which the user presses the personal care treatment unit against the body part, and wherein the pressure or force sensor is integrated in the coupling structure. In these embodiments the pressure or force sensor is used to detect whether the personal care device is in an enabled treatment condition, i.e., a condition wherein the personal care treatment unit is in sufficient contact with the body part. In particular, contact of the personal care treatment unit with the body part may be determined to be sufficient for the personal care treatment if the measured pressure or force is above a predefined threshold value. A user may inadvertently disable the pressure or force sensor from properly measuring the enabled treatment condition, e.g., by holding the personal care treatment unit in his or her hand instead of the main housing. In such a case, because the pressure or force sensor is integrated in the coupling structure between the main housing and the personal care treatment unit, the pressure or force sensor cannot properly measure the pressure or force with which the user presses the personal care treatment unit against the body part. For such a pressure or force sensor it is hardly or not possible to distinguish, based on the second sensor data, the disabled condition of the pressure or force sensor from the disabled treatment condition of the personal care device measured in the enabled condition of the pressure or force sensor.

In further embodiments of the personal care device and the personal care system according to the invention, the first sensor comprises one or more of a position sensor, a motion or displacement sensor, a velocity sensor, an acceleration sensor, an orientation sensor, a gyroscope, a magnetometer, an inertial measurement unit (IMU), a force or pressure sensor, a temperature sensor, and a timer. In these embodiments, the treatment data determined based on the first sensor data may, e.g., comprise a momentary location of the personal care device on the body part, a sequence of locations of the personal care device on the body part during a treatment session, or a dwell time of the personal care device for each of a plurality of segments of the body part during a treatment session. Treatment data involving a sequence of locations of the personal care device on the body part may further comprise data about other parameters measured for each location, such as measured pressure.

A preferred embodiment of the computer-implemented method according to the invention comprises, if the second sensor is determined to be in the disabled condition, providing feedback to a user of the personal care device relating to the disabled condition of the second sensor by providing a feedback instruction relating to the disabled condition to a feedback unit of the personal care device. Preferred embodiments of the personal care device and the personal care system according to the invention comprise a feedback unit, and in these embodiments the processor is configured to provide, if the second sensor is determined to be in the disabled condition, a feedback instruction to the feedback unit corresponding to feedback relating to the disabled condition of the second sensor. In these embodiments the user of the personal care device may be informed about the disabled condition of the second sensor by the feedback unit and may be advised to resolve the disabled condition of the second sensor. In embodiments of the personal care system, the feedback unit may be comprised by the personal care device of the personal care system or by a separate device of the personal care system, such as a smart phone or a separate display device.

A further embodiment of the computer-implemented method according to the invention comprises providing feedback to a user of the personal care device relating to the determined treatment data by providing a feedback instruction relating to the determined treatment data to a feedback unit of the personal care device. Further embodiments of the personal care device and the personal care system according to the invention comprise a feedback unit, and in these further embodiments the processor is further configured to provide a feedback instruction to the feedback unit corresponding to feedback relating to the determined treatment data. In these embodiments the user of the personal care device may be informed about the treatment data determined based on the first sensor data. In embodiments of the personal care system, the feedback unit may be comprised by the personal care device of the personal care system or by a separate device of the personal care system, such as a smart phone or a separate display device.

A still further embodiment of the computer-implemented method as claimed in any of the preceding claims comprises adjusting an operational parameter of the personal care device based on the determined treatment data. In still further embodiments of the personal care device and the personal care system according to the invention, the processor is configured to adjust an operational parameter of the personal care device based on the determined treatment data. In these embodiments the operation of the personal care device is automatically adjusted in dependence on the determined treatment data. Thus, the personal care treatment may be more efficient and effective.

According to a fourth aspect of the invention, a computer program product is provided comprising a non-transitory computer-readable medium, the computer-readable medium having computer-readable code embodied therein, the computer readable code being configured such that, on execution by a suitable computer or processor, the computer or processor is caused to perform the method as claimed in any of the preceding claims.

The above-described and other aspects of the invention will be apparent from and elucidated with reference to the following detailed description of embodiments of a computer-implemented method, a personal care device and a personal care system in accordance with the invention.

### BRIEF DESCRIPTION OF THE DRAWINGS

The invention will be explained in greater detail with reference to the Figures, in which equal or similar features are indicated by the same reference signs, and in which:
Fig. 1 schematically shows an embodiment of a personal care device in accordance with the invention;
Fig. 2 is a flowchart of a first embodiment of a computer-implemented method in accordance with the invention for determining treatment data relating to treatment of a body part of a subject by means of the personal care device of Fig. 1;
Fig. 3 schematically shows an embodiment of a personal care system in accordance with the invention; and
Fig. 4 is a flowchart of a second embodiment of a computer-implemented method in accordance with the invention for determining treatment data relating to treatment of a body part of a subject by means of the personal care system of Fig. 3.

### DETAILED DESCRIPTION OF THE EMBODIMENTS

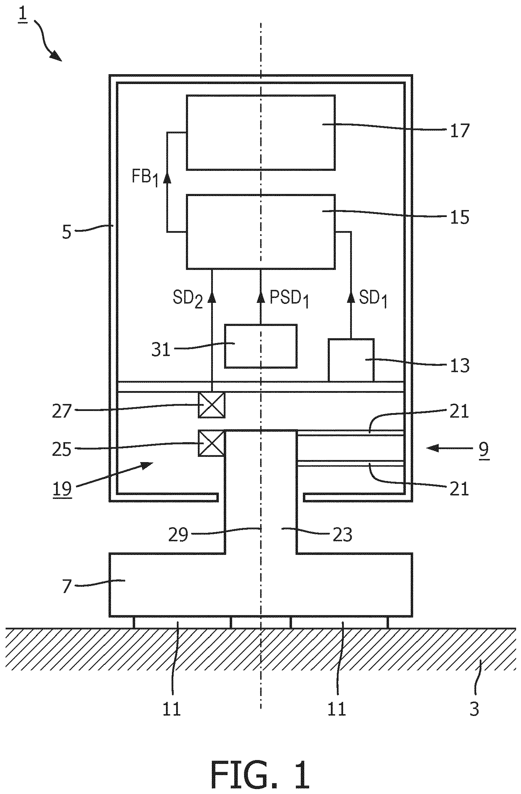

Fig. 1 schematically shows an embodiment of a personal care device 1 in accordance with the invention, configured to provide a personal care treatment to a body part 3 of a subject. The personal care device 1 comprises a main housing 5, configured to be handheld by a user, and a personal care treatment unit 7 coupled to the main housing 5 by means of a coupling structure 9. The personal care treatment unit 7 is configured to provide the personal care treatment to the body part 3. In the embodiment schematically shown in Fig. 1 the personal care device 1 is an electric shaver, the body part 3 is a beard area of the face of the user, and the personal care treatment unit 7 is a shaving unit comprising at least two hair-cutting units 11 configured to cut hairs present on the user's face. Electric shavers are well known to the skilled person, and the skilled person is aware of technical details of such an electric shaver, the shaving unit and the hair-cutting units 11 to enable a shaving process by means of the electric shaver.

In other embodiments of the invention, the personal care device may be of a different type configured to provide a personal care treatment to the body part of the subject different from a shaving process. Examples are hair-cutting devices other than electric shavers such as a hair trimmer, skin treatment devices such as a facial cleansing brush, a skin rejuvenation device or an intense pulsed light (IPL) device, and oral care devices such as a power toothbrush or an air flossing device. The term "body part" as used herein is intended to refer to any part of the body of a human that may be treated using a personal care device. For example, in the embodiment of an intense pulsed light (IPL) device the body part may be a leg of the subject on which a hair removal treatment is performed by means of the IPL device. In the embodiment of an oral care device the body part may comprise the teeth or gum of the subject on which an oral care treatment is performed by means of the oral care device.

The personal care device 1 shown in Fig. 1 comprises a first sensor 13 configured to generate first sensor data SD₁ relating to a treatment parameter of the personal care treatment at a first moment of time t₁. The personal care device 1 further comprises a processor 15 configured to receive the first sensor data SD₁ generated by the first sensor 13 and to determine, based on the first sensor data SD₁, treatment data relating to the personal care treatment. In the embodiment of the electric shaver shown in Fig. 1, the first sensor 13 comprises an inertial measurement unit (IMU), and the processor 15 is configured to determine, based on the first sensor data SD₁ generated by the IMU, a position of the electric shaver relative to the body part 3 during a shaving session or a sequence of positions of the electric shaver relative to the body part 3 during a shaving session.

Instead of an IMU, the first sensor 13 may comprise another type of position sensor, a motion sensor, a displacement sensor, a velocity sensor, an acceleration sensor, an orientation sensor, a gyroscope, a magnetometer, or a combination of two or more of such sensors by means of which the processor 15 can determine the position or the sequence of positions of the electric shaver relative to the body part 3 during a shaving session. The processor 15 may be configured, e.g., programmed to execute any known method to determine said location or sequence of locations based on the first sensor data SD₁, for example a method as disclosed by EP3938155B1 in the name of the applicant. The determined treatment data about the location of the electric shaver may be combined with first sensor data relating to another treatment parameter of the personal care treatment, such as a force or pressure exerted by the personal care treatment unit 7 on the body part 3 measured by a force or pressure sensor, a temperature of the body part 3 measured by a temperature sensor, or timing information measured by a timer. In this manner, the treatment data determined by the processor 15 based on the first sensor data SD₁ may, e.g., comprise information about the measured force or pressure or the measured temperature as a function of the location of the electric shaver relative to the body part 3 or information about the dwell time of the electric shaver for each of a plurality of segments of the body part 3 during a shaving session.

The personal care device 1 further comprises a feedback unit 17 schematically shown in Fig. 1. The feedback unit 17 may comprise a display arranged on the main housing 5. The processor 15 is further configured to provide a first feedback instruction FB₁ to the feedback unit 17 corresponding to feedback relating to the treatment data determined by the processor 15 based on the first sensor data SD₁. Thus, the user of the personal care device 1 may be informed, via the feedback unit 17, about the treatment data determined by the processor 15.

Additionally or alternatively to providing the first feedback instruction FB₁ to the feedback unit 17, the processor 15 may be configured to adjust an operational parameter of the personal care device 1 based on the determined treatment data. In the example of an electric shaver as shown in Fig. 1, the treatment data determined by the processor 15 based on the first sensor data SD₁ may comprise the location of the electric shaver relative to the body part 3, and the processor 15 may be configured to adjust, e.g., a speed of a motor of the electric shaver, arranged to drive the hair-cutting units 11, in dependence on the determined location of the electric shaver relative to the body part 3.

For the determination of the treatment data by the processor 15 based on the first sensor data SD₁, it may be useful to disregard first sensor data SD₁ which are generated by the first sensor 13 during periods of a treatment session wherein the personal care device 1 is disabled to perform the personal care treatment, e.g., when the user has not yet started the shaving process or has paused the shaving process by moving the electric shaver away from the body part 3. For this purpose, the personal care device 1 has a second sensor 19 configured to generate second sensor data SD₂ relating to a treatment condition of the personal care device 1 with respect to the body part 3 at said first moment of time ti, wherein the treatment condition indicates whether the personal care treatment by the personal care device 1 is enabled at said first moment of time t₁. The processor 15 is configured to receive the second sensor data SD₂ and to process the second sensor data SD₂ to determine whether, at said first moment of time ti, the personal care device 1 is in an enabled treatment condition with respect to the body part 3, wherein the enabled treatment condition enables the personal care treatment. If, based on the second sensor data SD₂, the processor 15 determines that the personal care device 1 is in the enabled treatment condition at said first moment of time ti, the processor 15 determines the treatment data based on the first sensor data SD₁ generated by the first sensor 13 and relating to the treatment parameter measured at said first moment of time t₁. In other words, in that case the processor 15 takes into account the first sensor data SD₁ generated for said first moment of time t₁ when determining the treatment data.

In the example of the electric shaver schematically shown in Fig. 1, the treatment condition measured by the second sensor 19 is physical contact of the personal care device 1 (electric shaver) with the body part 3. In particular, the enabled treatment condition of the personal care device 1 (electric shaver) involves a condition wherein the personal care treatment unit 7 (shaving unit) is in contact with the body part 3. To detect contact of the personal care treatment unit 7 (shaving unit) with the body part 3, the second sensor 19 comprises a pressure sensor configured to detect a pressure with which the user presses the personal care treatment unit 7 (shaving unit) against the body part 3. If the pressure measured by the second sensor 19 exceeds a predefined threshold value, the processor 15 determines that the personal care device 1 (electric shaver) is in the enabled treatment condition. In the example schematically shown in Fig. 1, the pressure sensor is of a type as disclosed by EP3852983B1 in the name of applicant, wherein the pressure sensor is integrated in the coupling structure 9 by means of which the personal care treatment unit 7 (shaving unit) is coupled to the main housing 5. In particular, as schematically shown in Fig. 1, the coupling structure 9 comprises two parallel blade springs 21 by means of which a central coupling member 23 of the personal care treatment unit 7 (shaving unit) is elastically suspended relative to the main housing 5. The second sensor 19 comprises a magnet 25, mounted in a fixed position to the central coupling member 23, and a Hall sensor 27 mounted in a fixed position within and relative to the main housing 5. Under influence of pressure forces exerted by the body part 3 on the hair-cutting units 11, the personal care treatment unit 7 (shaving unit) is displaceable relative to the main housing 5, by elastic deformation of the blade springs 21, in an axial direction parallel to a central axis 29 of the personal care treatment unit 7. An amount of said displacement of the personal care treatment unit 7 depends on said pressure forces and on the known elastic properties of the blade springs 21. The Hall sensor 27 of the second sensor 19 is arranged to measure said displacement of the personal care treatment unit 7 relative to the main housing 5, and the processor 15 is configured to determine the treatment condition of the personal care device 1 based on said displacement of the personal care treatment unit 7 measured by the second sensor 19, taking into account the known elastic properties of the blade springs 21 and the predefined threshold value for the pressure mentioned here before.

If, based on the second sensor data SD₂ generated by the second sensor 19 at said first moment of time t₁ during a shaving session, the processor 15 determines that the personal care device 1 (electric shaver) is not in an enabled treatment condition with respect to the body part 3, i.e., in case of no contact of the personal care treatment unit 7 (shaving unit) with the body part 3, the processor 15 could just disregard the first sensor data SD₁ generated by the first sensor 13 at said first moment of time t₁ when determining the treatment data relating to the personal care treatment. I.e., in such case the processor 15 could just not take the first sensor data SD₁ generated at said first moment of time t₁ into account when determining the treatment data. However, such a general approach would not take into account the fact that the user may inadvertently disable the second sensor 19 from properly measuring the treatment condition of the personal care device 1 with respect to the body part 3. When the second sensor 19 is disabled, the second sensor data SD₂ may, e.g., indicate that the personal care device 1 is not in the enabled treatment condition also when the personal care device 1 actually is in the enabled treatment condition. In such a case, according to the general approach described here before the first sensor data SD₁, generated during periods wherein the second sensor 19 is disabled, could be wrongly disregarded when determining the treatment data, so that the treatment data may become inaccurate or at least incomplete.

According to the present invention, the disadvantages resulting from a general approach as described here before are at least alleviated to some extent in that the processor 15 is configured to compare the second sensor data SD₂ with first predetermined sensor data PSD₁ which are characteristic of a disabled condition of the second sensor 19 wherein the second sensor 19 is disabled from measuring the treatment condition of the personal care device 1 with respect to the body part 3. As schematically shown in Fig. 1, the first predetermined sensor data PSD₁ may be stored in a memory 31 connected with the processor 15. The processor 15 is further configured to determine, based on comparing the first sensor data SD₁ generated at said first moment of time t₁ with the first predetermined sensor data PSD₁, whether the second sensor 19 is in the disabled condition at said first moment of time t₁. If the processor 15 determines that, at said first moment of time ti, the second sensor 19 is in the disabled condition, the processor 15 determines, based on a predefined data usage rule, whether to base the treatment data on the first sensor data SD₁ generated at said first moment of time ti, i.e., whether to take the first sensor data SD₁ generated at said first moment of time t₁ into account when determining the treatment data.

In the embodiment of the personal care device 1 (electric shaver) according to the invention shown in Fig. 1, a user may inadvertently disable the second sensor 19 from properly measuring the enabled treatment condition by holding, during the shaving process, the personal care treatment unit 7 (shaving unit) in his or her hand instead of the main housing 5. Investigations have shown that, for the specific type of pressure sensor (second sensor 19) as shown in Fig. 1, this is the most common way in which the user may inadvertently disable the second sensor 19. Because the second sensor 19 is integrated in the coupling structure 9 between the main housing 5 and the personal care treatment unit 7 (shaving unit), the pressure measured by the second sensor 19 when the user holds the personal care treatment unit 7 (shaving unit) in his or her hand during the shaving process is close to zero, i.e., the measured pressure is below the predefined threshold value as mentioned here before. As a result, the pressure measured by the second sensor 19 in its disabled condition is similar to the pressure measured by the second sensor 19, in its enabled condition, when the personal care device 1 (electric shaver) is not in the enabled treatment condition. In other words, based on the second sensor data SD₂, the processor 15 cannot distinguish the disabled condition of the second sensor 19 from the disabled treatment condition of the personal care device 1 measured in the enabled condition of the second sensor 19.

In the embodiment of Fig. 1, the predefined data usage rule, based on which the processor 15 determines whether to take into account, when determining the treatment data, the first sensor data SD₁ for moments of time at which the second sensor 19 is determined to be in the disabled condition, is based on the assumption that, if a user disables the second sensor 19 during a relatively long portion of a shaving session, the personal care device 1 (electric shaver) will most likely be in the enabled treatment condition for a larger part of said portion of the shaving session during which the second sensor 19 is disabled. Accordingly, based on said assumption, it makes most sense to base the treatment data also on the first sensor data SD₁ received during said period. However, when the disabled condition of the second sensor 19 is determined during only a relatively short portion of a shaving session, the disabled condition of the second sensor 19 most likely represents periods during which the personal care device 1 (electric shaver) is in the disabled treatment condition not in contact with the body part 3. Accordingly, in such case it makes most sense to base the treatment data only on the first sensor data SD₁ received during periods wherein the second sensor 19 measures the enabled treatment condition of the personal care device 1. It will be evident for the skilled person that a predefined data usage rule, which is based on the above mentioned assumptions, needs to be applied by the processor 15 after completion of a shaving session when the second sensor data SD₂ are available for each moment of time within the shaving session, or after receipt of at least the first and second sensor data SD₁, SD₂ for a plurality of moments of time.

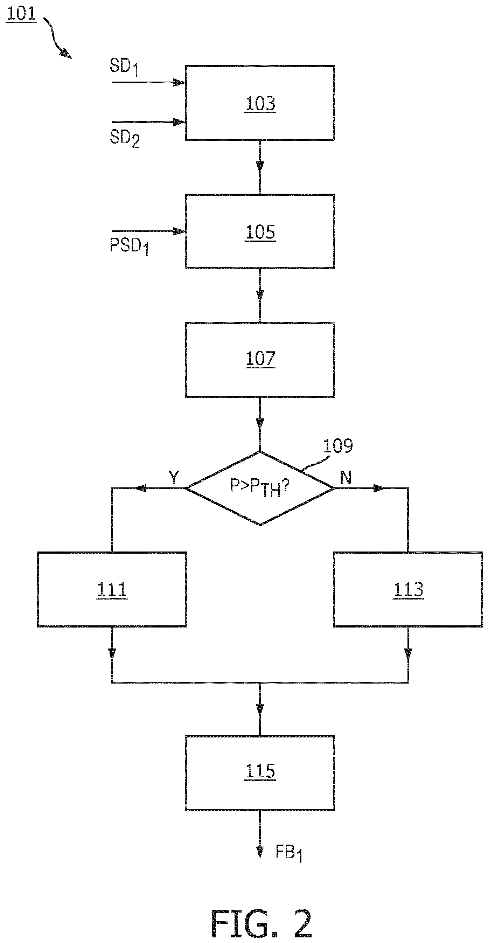

For this purpose, the processor 15 is configured, e.g., programmed, to execute a first embodiment of a computer-implemented method 101 according to the invention for determining the treatment data relating to the treatment of the body part 3 by means of the personal care device 1, as illustrated by the flowchart shown in Fig. 2. In a first step 103 of the method 101, the processor 15 receives a plurality of the first sensor data SD₁ and a plurality of the second sensor data SD₂ relating to, respectively, the treatment parameter and the treatment condition of the personal care device 1 (electric shaver) at a plurality of successive moments of time during a shaving session. In a second step 105 of the method 101, the processor 15 determines for each moment of time of said plurality of successive moments of time, based on comparing the second sensor data SD₂ with the first predetermined sensor data PSD₁, whether the second sensor 19 is in the disabled condition. The second step 105 may be executed simultaneously with the first step 103, i.e., the second sensor data SD₂ received for a particular moment of time in the first step 103 may be compared with the first predetermined sensor data PSD₁ immediately after receipt of said second sensor data SD₂. Alternatively, the second step 105 may be executed after the first step 103, i.e., after receipt of the plurality of second sensor data SD₂. In a third step 107 of the method 101, executed after receiving said plurality of the first sensor data SD₁ and said plurality of the second sensor data SD₂, i.e., after the first and second steps 103, 105, the processor 15 determines, based on the results of comparing the second sensor data SD₂ with the first predetermined sensor data PSD₁ in step 105, a portion P of said plurality of successive moments of time for which the second sensor 19 is determined to be in the disabled condition.

In the first embodiment of the computer-implemented method 101, the predefined data usage rule causes the processor 15 to base the treatment data on the first sensor data SD₁, received for each moment of time of said plurality of successive moments of time, if said determined portion P of said plurality of successive moments of time is above a predefined threshold P_{TH}. Furthermore, the predefined data usage rule causes the processor 15 to base the treatment data on only the first sensor data SD₁, received for the moments of time of said plurality of successive moments of time for which the second sensor 19 is determined not to be in the disabled condition, if said determined portion P of said plurality of successive moments of time is below said predefined threshold P_{TH}. A suitable value for said predefined threshold P_{TH} is 15%. For this purpose, in a fourth step 109 of the method 101 the processor 15 determines whether the portion P determined in the third step 107 is above said predefined threshold P_{TH}. If in the fourth step 109 the processor 15 determines that the portion P is above said predefined threshold P_{TH}, the processor 15 bases, in a fifth step 111 of the method 101, the treatment data on the first sensor data SD₁ received for each moment of time of said plurality of successive moments of time. If in the fourth step 109 the processor 15 determines that the portion P is below or equal to said predefined threshold P_{TH}, the processor 15 bases, in a sixth step 113 of the method 101, the treatment data on only the first sensor data SD₁ received for the moments of time of said plurality of successive moments of time for which the second sensor 19 is determined not to be in the disabled condition. Finally, in a seventh step 115 of the method 101 the processor 15 provides the feedback instruction FB₁ relating to the determined treatment data to the feedback unit 17 of the personal care device 1, to inform the user of the personal care device 1 about the determined treatment data.

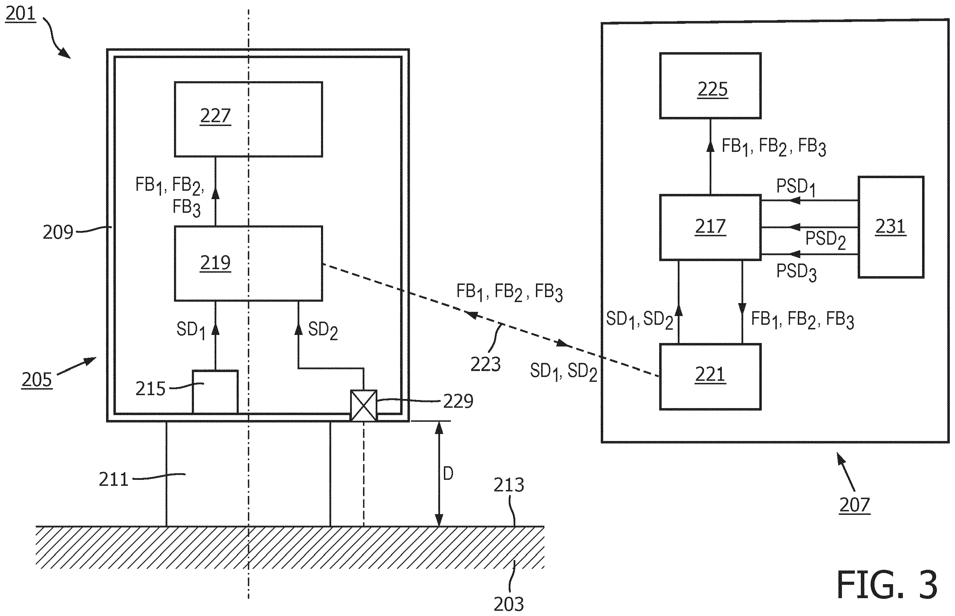

Fig. 3 schematically shows an embodiment of a personal care system 201 in accordance with the invention, configured to provide a personal care treatment to a body part 203 of a subject. The personal care system 201 comprises a personal care device 205 and a remote electronic processing device 207. The personal care device 205 comprises a main housing 209, configured to be handheld by a user, and a personal care treatment unit 211 connected to the main housing 209. The personal care treatment unit 211 is configured to provide the personal care treatment to the body part 203 when the personal care treatment unit 211 is in contact with a surface 213 of the body part 203. In the embodiment schematically shown in Fig. 3, the personal care device 205 is a light-based skin treatment device, the body part 203 is, e.g., a leg of the user, the surface 213 is, e.g., a skin surface of the body part 203, and the personal care treatment unit 211 is a light module configured to provide light to the body part 203, e.g., to remove hairs from the skin surface of the body part 203 or to treat particular skin disorders. Light-based skin treatment devices are well known to the skilled person, and the skilled person is aware of technical details of such a light-based skin treatment device and the light module to enable a light-based skin treatment, such as hair removal, by means of the light-based skin treatment device. As described here before with respect to the embodiment of the personal care device 1 in accordance with the invention, in other embodiments of the personal care system the personal care device of the personal care system may be of a different type configured to provide a personal care treatment to the body part of the subject different from a light-based skin treatment.

The personal care device 205 of the personal care system 201 shown in Fig. 3 comprises a first sensor 215 configured to generate first sensor data SD₁ relating to a treatment parameter of the personal care treatment at a first moment of time t₁. The remote electronic processing device 207 of the personal care system 201 comprises a processor 217 configured to receive the first sensor data SD₁ generated by the first sensor 215 and to determine, based on the first sensor data SD₁, treatment data relating to the personal care treatment. In the embodiment of the personal care system 201 with the light-based skin treatment device shown in Fig. 3, the first sensor 215 comprises an inertial measurement unit (IMU), and the processor 217 is configured to determine, based on the first sensor data SD₁ generated by the IMU, a position of the personal care device 205 (light-based skin treatment device) relative to the body part 203 during a treatment session or a sequence of positions of the personal care device 205 relative to the body part 203 during the treatment session. As described here before with respect to the embodiment of the personal care device 1 in accordance with the invention, in other embodiments of the personal care system, instead of an IMU, the first sensor 215 may comprise another type of sensor or a combination of sensors by means of which the processor 217 can determine the position or the sequence of positions of the personal care device 205 relative to the body part 203 during a treatment session, and the determined treatment data about the location of the personal care device 205 may be combined with first sensor data relating to another treatment parameter of the personal care treatment.

The electronic processing device 207 may be a smartphone, laptop, smartwatch, computer or any other remote computing device such as a computer of a cloud server. To enable the processor 217 of the electronic processing device 207 to receive the first sensor data SD₁ generated by the first sensor 215 of the personal care device 205, the personal care device 205 comprises first interface circuitry 219 and the electronic processing device 207 comprises second interface circuitry 221. First and second interface circuitry 219, 221 are configured to enable a data connection and/or data exchange 223 between the personal care device 205 and the remote electronic processing device 207. Said data connection and/or data exchange 223 may be direct between first and second interface circuitry 219, 221 or indirect, e.g., via the internet. A direct data connection and/or data exchange 223 may be provided by any known wired or wireless communication protocol, such as Wi-Fi, Bluetooth, Zigbee, or any cellular communication protocol including but not limited to Global System for Mobile Communications (GSM), Universal Mobile Telecommunications System (UMTS), Long Term Evolution (LTE), LTE-Advanced, etc.. In case of a direct wireless connection, first and second interface circuitry 219, 221 may each include a suitable antenna for transmitting and/or receiving data over a transmission medium, e.g., air. The first interface circuitry 219 is connected to the first sensor 215 for receiving the first sensor data SD₁ and transmitting the first sensor data SD₁ to the second interface circuitry 219 via data connection 223. The processor 217 is connected to the second interface circuitry 219 to receive the first sensor data SD₁ transmitted via the data connection 223.

The electronic processing device 207 further comprises a feedback unit 225 schematically shown in Fig. 3. The feedback unit 225 may, e.g., comprise a display, e.g., a smartphone display in case the electronic processing device 207 is a smartphone. The processor 217 is configured to provide a first feedback instruction FB₁ to the feedback unit 225 corresponding to feedback relating to the treatment data determined by the processor 217 based on the first sensor data SD₁. Thus, the user of the personal care system 207 may be informed, via the feedback unit 225 of the remote electronic processing device 207, about the treatment data determined by the processor 207. In addition or alternatively, the processor 217 may be configured to provide the first feedback instruction FB₁ to a feedback unit 227 of the personal care device 205, i.e. via first and second interface circuitry 219, 221 and data connection 223.

Additionally or alternatively to providing the first feedback instruction FB₁, the processor 217 may be configured to adjust an operational parameter of the personal care device 205 based on the determined treatment data. In the example of the light-based skin treatment device as shown in Fig. 3, the treatment data determined by the processor 217 may comprise the location of the personal care device 205 relative to the body part 203, and the processor 217 may be configured to adjust, e.g., a light intensity of the personal care treatment unit 211 (light module) in dependence on the determined location of the personal care device 205 relative to the body part 203.

Like the personal care device 1 as described here before, the personal care device 205 of the personal care system 201 shown in Fig. 3 has a second sensor 229 configured to generate second sensor data SD₂ relating to a treatment condition of the personal care device 205 with respect to the body part 203 at said first moment of time ti, wherein the treatment condition indicates whether the personal care treatment by the personal care device 205 is enabled at said first moment of time t₁. The processor 217 is configured to receive, via first and second interface circuitry 219, 221 and data connection 223, the second sensor data SD₂ and to process the second sensor data SD₂ to determine whether, at said first moment of time ti, the personal care device 205 is in an enabled treatment condition with respect to the body part 203, wherein the enabled treatment condition enables the personal care treatment. If the processor 217 determines that the personal care device 205 is in the enabled treatment condition at said first moment of time ti, the processor 217 determines the treatment data based on the first sensor data SD₁ generated by the first sensor 215 and relating to the treatment parameter measured at said first moment of time t₁.

In the example of the light-based skin treatment device schematically shown in Fig. 3, the treatment condition measured by the second sensor 229 is physical contact of the personal care device 205 with the body part 203. In particular, the enabled treatment condition of the personal care device 205 involves a condition wherein the personal care treatment unit 211 (light module) is in contact with the surface 213 of the body part 203. To detect contact of the personal care treatment unit 211 (light module) with the surface 213 of the body part 203, the second sensor 229 comprises a proximity sensor arranged in the main housing 209 of the personal care device 205 such that, in the enabled treatment condition with the personal care treatment unit 211 (light module) in contact with the surface 213 of the body part 203, the proximity sensor is at a predefined distance D from the surface 213 of the body part 203, as shown in Fig. 3. If the proximity sensor detects the presence of the surface 213 of the body part 203 at said predefined distance D, the processor 217 determines, based on the second sensor data SD₂, that the personal care device 205 is in the enabled treatment condition. The proximity sensor may, e.g., be an infrared photoelectric proximity sensor.

In the embodiment of the personal care system 201 according to the invention shown in Fig. 3, a user may inadvertently disable the second sensor 229 from properly measuring the enabled treatment condition of the personal care device 205 by holding a finger against the second sensor 229 or between the second sensor 229 and the surface 213 of the body part 203 during the treatment process. Investigations have shown that, for a proximity sensor arranged in the personal care device 205 in the manner shown in Fig. 3, this is the most common way in which the user may inadvertently disable the second sensor 229. Contrary to the second sensor 19 (pressure sensor) of the embodiment of the personal care device 1 as described here before, for the second sensor 229 (proximity sensor) it is possible to distinguish, based on the second sensor data SD₂, with sufficient accuracy the disabled condition of the second sensor 229 from the disabled treatment condition of the personal care device 205 measured in the enabled condition of the second sensor 229. In particular, in the enabled condition of the second sensor 229, the second sensor 229 will measure a distance larger than the predefined distance D if the personal care device 205 is in the disabled treatment condition. In the disabled condition of the second sensor 229, the second sensor 229 will measure a distance substantially smaller than the predefined distance D when the user holds a finger before the second sensor 229, or the second sensor 229 is completely disabled from measuring when the user holds a finger against the second sensor 229. Because, based on the second sensor data SD₂, the processor 217 can distinguish the disabled condition of the second sensor 229 from the disabled treatment condition of the personal care device 205 measured in the enabled condition of the second sensor 229, the processor 217 is configured to disregard, when determining the treatment data, the first sensor data SD₁ received for those moments of time for which the personal care device 205 is determined to be in the disabled treatment condition. Furthermore, in the embodiment of the personal care system 201, according to the predefined data usage rule the treatment data are also based on the first sensor data SD₁ received for those moments of time for which the second sensor 229 is determined to be in the disabled condition. In the embodiment of the personal care system 201 having the proximity sensor, the predefined data usage rule is based on the assumption that, if the user disables the second sensor 229 (proximity sensor) during a treatment session, it is most likely that the personal care device 205 will be in the enabled treatment condition with respect to the body part 203, so that it makes most sense to also include the first sensor data SD₁, received when the second sensor 229 is in the disabled condition, when determining the treatment data. It will be evident for the skilled person that, in the embodiment of the personal care system 201, the treatment data may be determined based on the first sensor data SD₁ in real-time during a treatment session.

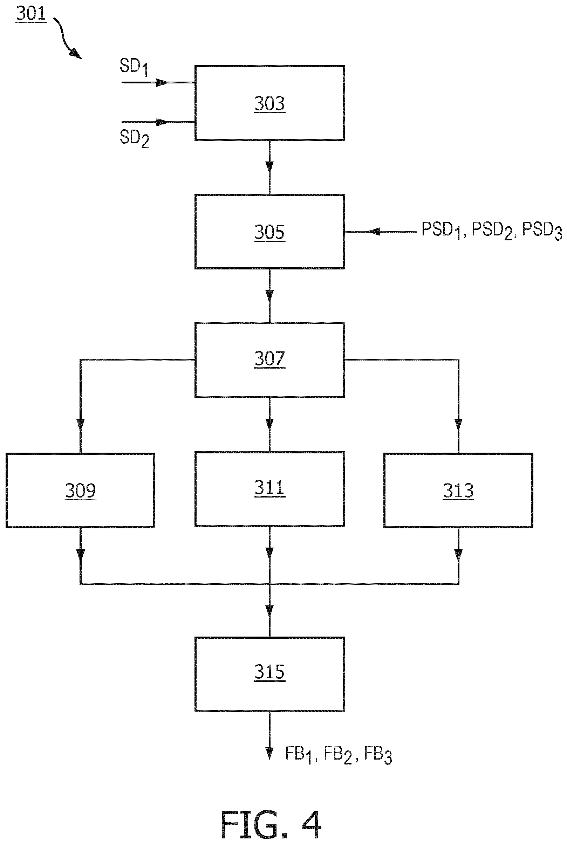

For this purpose, the processor 217 is configured, e.g., programmed, to execute a second embodiment of a computer-implemented method 301 according to the invention for determining the treatment data relating to the treatment of the body part 203 by means of the personal care device 205, as illustrated by the flowchart shown in Fig. 4. In a first step 303 of the method 301, the processor 217 receives the first sensor data SD₁ generated by the first sensor 215 at a first moment of time t₁ and the second sensor data SD₂ generated by the second sensor 229 at said first moment of time t₁. The first and second sensor data SD₁, SD₂ are transmitted from the personal care device 205 to the remote electronic processing device 207 by first and second interface circuitry 219, 221, as shown in Fig. 3. In a second step 305 of the method 301, the processor 217 compares the second sensor data SD₂ with first predetermined sensor data PSD₁, second predetermined sensor data PSD₂ and third predetermined sensor data PSD₃. The first predetermined sensor data PSD₁ are characteristic of the disabled condition of the second sensor 229 as described here before. The second and third predetermined sensor data PSD₂, PSD₃ are both characteristic of the enabled condition of the second sensor 229 as described here before, wherein the second predetermined sensor data PSD₂ are characteristic of the enabled treatment condition of the personal care device 205 as described here before, and wherein the third predetermined sensor data PSD₃ are different from the first predetermined sensor data, as described here before, and characteristic of the disabled treatment condition of the personal care device 205. As schematically shown in Fig. 3, the first, second and third predetermined sensor data PSD₁, PSD₂ and PSD₃ may be stored in a memory 231 of the electronic processing device 207 which is connected with the processor 217.

In a third step 307 of the method 301, the processor 217 determines, based on the results of comparing the second sensor data SD₂ with the first predetermined sensor data PSD₁ in the second step 305, whether the second sensor 229 is in the disabled condition or in the enabled condition at said first moment of time t₁. If the second sensor 229 is determined to be in the enabled condition, the processor 217 determines in the third step 307, based on the results of comparing the second sensor data SD₂ with the second and third predetermined sensor data PSD₂, PSD₃ in the second step 305, whether the personal care device 205 is in the enabled treatment condition or in the disabled treatment condition at said first moment of time t₁.

If in the third step 307 of the method 301 the processor 217 determines that the personal care device 205 is in the enabled treatment condition at said first moment of time ti, the processor 217 determines, in a fourth step 309 of the method 301, the treatment data based on the first sensor data SD₁ generated by the first sensor 215 at said first moment of time t₁. If in the third step 307 of the method 301 the processor 217 determines that the personal care device 205 is in the disabled treatment condition at said first moment of time ti, the processor 217 disregards, in a fifth step 311 of the method 301, the first sensor data SD₁ generated by the first sensor 215 at said first moment of time t₁ when determining the treatment data. If in the third step 307 of the method 301 the processor 217 determines that the second sensor 229 is in the disabled condition at said first moment of time ti, the processor 217 bases, in a sixth step 313 of the method 301, the treatment data also on the first sensor data SD₁ generated by the first sensor 215 at said first moment of time t₁ in accordance with the predefined data usage rule for the embodiment of the personal care system 201 as described here before.

In a seventh step 315 of the method 301, the processor 217 provides the feedback instruction FB₁, relating to the treatment data determined in the fourth, fifth and sixth steps 309, 311, 313 of the method 301, to the feedback unit 225 of the electronic processing device 207 to inform the user of the personal care system 201 about the determined treatment data. In addition or alternatively, the processor 217 may provide in the seventh step 315 the first feedback instruction FB₁ to the feedback unit 227 of the personal care device 205, i.e. via first and second interface circuitry 219, 221 as shown in Fig. 3. If in the third step 307 of the method 301 the processor 217 determines that the second sensor 229 is in the disabled condition at said first moment of time ti, the processor 217 may, in the seventh step 315, further provide a second feedback instruction FB₂ corresponding to feedback relating to the disabled condition of the second sensor 229. The second feedback instruction FB₂ is preferably provided to the feedback unit 227 of the personal care device 205, i.e. via first and second interface circuitry 219, 221 as shown in Fig. 3, so that the user of the personal care device 205 is immediately informed about the disabled condition of the second sensor 229 during the treatment process. The second feedback instruction FB₂ may include an instruction for the user to prevent the disabled condition of the second sensor 229. Of course, the second feedback instruction FB₂ may also be provided to the feedback unit 225 of the electronic processing device 207. Finally, if in the third step 307 of the method 301 the processor 217 determines that the personal care device 205 is in the disabled treatment condition at said first moment of time ti, the processor 217 may, in the seventh step 315, further provide a third feedback instruction FB₃ corresponding to feedback relating to the disabled treatment condition of the personal care device 205. The third feedback instruction may be provided to the feedback unit 227 of the personal care device 205, i.e. via first and second interface circuitry 219, 221 as shown in Fig. 3, or to the feedback unit 225 of the electronic processing device 207.

The processor 15, 217 as described here before can be implemented in numerous ways, with software and/or hardware, to perform the various functions as described here before. The processor 15, 217 may comprise one or more microprocessors or digital signal processors (DSPs) that may be programmed using software or computer program code to perform the required functions and/or to control components of the processor 15, 217 to achieve the required functions. The processor 15, 217 may be implemented as a combination of dedicated hardware to perform some functions (e.g., amplifiers, preamplifiers, analog-to-digital convertors (ADCs) and/or digital-to-analog convertors (DACs)) and a processing member (e.g., one or more programmed microprocessors, controllers, DSPs and associated circuitry) to perform other functions. Examples of components that may be employed in various embodiments of the invention include, but are not limited to, conventional microprocessors, DSPs, application specific integrated circuits (ASICs), and field-programmable gate arrays (FPGAs).

The invention also relates to computer program products, particularly computer programs on or in a carrier, adapted to put the invention into practice. The computer program may be in the form of a source code, an object code, a code intermediate source and an object code such as in a partially compiled form, or in any other form suitable for use in the implementation of the method according to embodiments of the invention. It will also be appreciated that such a computer program may have many different architectural designs. For example, a program code implementing the functionality of the method, device or system according to the invention may be sub-divided into one or more sub-routines. Many different ways of distributing the functionality among these sub-routines will be apparent to the skilled person. The sub-routines may be stored together in one executable file to form a self-contained computer program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main computer program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the computer program. For example, the carrier may include a data storage, such as a ROM, for example a CD ROM or a semiconductor ROM, or a magnetic recording medium, for example a hard disk. Furthermore, the carrier may be a transmissible carrier such as an electric or optical signal, which may be conveyed via electric or optical cable or by radio or other means. When the computer program is embodied in such a signal, the carrier may be constituted by such a cable or other device or means. Alternatively, the carrier may be an integrated circuit in which the computer program is embedded, the integrated circuit being adapted to perform, or being used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope of the claims.

## Claims

1. A computer-implemented method (101, 301) for determining treatment data relating to treatment of a body part (3, 203) of a subject by means of a personal care device (1, 205), the method comprising:
- receiving (103, 303) first sensor data (SD₁) generated by a first sensor (13, 215) associated with the personal care device and relating to a treatment parameter of the treatment at a first moment of time (ti);
- receiving (103, 303) second sensor data (SD₂) generated by a second sensor (19, 229) associated with the personal care device and relating to a treatment condition of the personal care device with respect to the body part indicating whether the treatment by the personal care device is enabled at said first moment of time;
- processing the second sensor data to determine whether, at said first moment of time, the personal care device is in an enabled treatment condition with respect to the body part wherein the treatment is enabled; and
- if the personal care device is determined to be in the enabled treatment condition at said first moment of time, determining (309) the treatment data based on the first sensor data relating to the treatment parameter at said first moment of time;
**characterized in that** the method (101, 301) further comprises:
- comparing (105, 305) the second sensor data (SD₂) with first predetermined sensor data (PSD₁) characteristic of a disabled condition of the second sensor (19, 229) wherein the second sensor is disabled from measuring the treatment condition of the personal care device (1, 205) with respect to the body part (3, 203);
- determining (105, 307), based on said comparing, whether the second sensor is in the disabled condition at said first moment of time (ti); and
- if the second sensor is determined to be in the disabled condition at said first moment of time, determining (109, 111, 113; 313), based on a predefined data usage rule, whether to base the treatment data on the first sensor data (SD₁) relating to the treatment parameter at said first moment of time.

2. The computer-implemented method (101) as claimed in claim 1, wherein the method comprises:
- receiving (103) a plurality of the first sensor data (SD₁) and a plurality of the second sensor data (SD₂) relating to, respectively, the treatment parameter and the treatment condition of the personal care device (1) at a plurality of successive moments of time during a treatment session;
- determining (105) for each moment of time of said plurality of successive moments of time, based on said comparing, whether the second sensor (19) is in the disabled condition; and
- after receiving said plurality of the first sensor data and said plurality of the second sensor data, determining (107) a portion (P) of said plurality of successive moments of time for which the second sensor is determined to be in the disabled condition;
and wherein the predefined data usage rule causes the method to base (111, 113) the treatment data on:
- the first sensor data received for each moment of time of said plurality of successive moments of time if said determined portion is above a predefined threshold (P_{TH}); and
- only the first sensor data received for the moments of time of said plurality of successive moments of time for which the second sensor is determined not to be in the disabled condition if said determined portion is below said predefined threshold.

3. The computer-implemented method (301) as claimed in claim 1, wherein the method comprises:
- comparing (305) the second sensor data (SD₂) with second and third predetermined sensor data (PSD₂, PSD₃) characteristic of an enabled condition of the second sensor (229) wherein the second sensor is enabled to measure the treatment condition of the personal care device (205) with respect to the body part (203), wherein the second predetermined sensor data (PSD₂) are characteristic of the enabled treatment condition of the personal care device, and wherein the third predetermined sensor data (PSD₃) are different from the first predetermined sensor data (PSD₁) and characteristic of a disabled treatment condition of the personal care device with respect to the body part wherein the treatment is not enabled;
- determining (307), based on said comparing of the second sensor data with said second and third predetermined sensor data, whether the personal care device is in the disabled treatment condition at said first moment of time (ti); and
- if the personal care device is determined to be in the disabled treatment condition at said first moment of time, disregarding (311) the first sensor data (SD₁) relating to the treatment parameter at said first moment of time when determining the treatment data;
and wherein the predefined data usage rule causes the method to base (313) the treatment data on the first sensor data (SD₁) relating to the treatment parameter at said first moment of time if the second sensor is determined to be in the disabled condition at said first moment of time.

4. The computer-implemented method (301) as claimed in any of the preceding claims, further comprising, if the second sensor (229) is determined to be in the disabled condition, providing feedback to a user of the personal care device (205) relating to the disabled condition of the second sensor by providing a feedback instruction (FB₂) relating to the disabled condition to a feedback unit (227) of the personal care device (205).

5. The computer-implemented method (101, 301) as claimed in any of the claims 1-3, further comprising providing feedback to a user of the personal care device (1, 205) relating to the determined treatment data by providing a feedback instruction (FB₁) relating to the determined treatment data to a feedback unit (17, 227) of the personal care device.

6. The computer-implemented method (101, 301) as claimed in any of the preceding claims, further comprising adjusting an operational parameter of the personal care device (1, 205) based on the determined treatment data.

7. A computer program product comprising a non-transitory computer-readable medium, the computer-readable medium having computer-readable code embodied therein, the computer readable code being configured such that, on execution by a suitable computer or processor (15, 217), the computer or processor is caused to perform the method (101, 301) as claimed in any of the preceding claims.

8. A personal care device (1) configured to provide a personal care treatment to a body part (3) of a subject, comprising:
- a first sensor (13) configured to generate first sensor data (SD₁) relating to a treatment parameter of the personal care treatment at a first moment of time (ti);
- a second sensor (19) configured to generate second sensor data (SD₂) relating to a treatment condition of the personal care device with respect to the body part indicating whether the personal care treatment by the personal care device is enabled at said first moment of time; and
- a processor (15) configured to:
- receive the first sensor data and the second sensor data;
- process the second sensor data to determine whether, at said first moment of time, the personal care device is in an enabled treatment condition with respect to the body part enabling the personal care treatment; and
- if the personal care device is determined to be in the enabled treatment condition at said first moment of time, determine treatment data relating to the personal care treatment based on the first sensor data relating to the treatment parameter at said first moment of time;
**characterized in that** the processor (15) is further configured to:
- compare the second sensor data (SD₂) with first predetermined sensor data (PSD₁) characteristic of a disabled condition of the second sensor (19) wherein the second sensor is disabled from measuring the treatment condition of the personal care device (1) with respect to the body part (3);
- determine, based on said comparing, whether the second sensor is in the disabled condition at said first moment of time (ti); and
- if the second sensor is determined to be in the disabled condition at said first moment of time, determine, based on a predefined data usage rule, whether to base the treatment data on the first sensor data (SD₁) relating to the treatment parameter at said first moment of time.

9. A personal care system (201) comprising:
- a personal care device (205) configured to provide a personal care treatment to a body part (203) of a subject;
- a first sensor (215) associated with the personal care device configured to generate first sensor data (SD₁) relating to a treatment parameter of the personal care treatment at a first moment of time (ti);
- a second sensor (229) associated with the personal care device configured to generate second sensor data (SD₂) relating to a treatment condition of the personal care device with respect to the body part indicating whether the personal care treatment by the personal care device is enabled at said first moment of time; and
- a processor (217) configured to:
- receive the first sensor data and the second sensor data;
- process the second sensor data to determine whether, at said first moment of time, the personal care device is in an enabled treatment condition with respect to the body part enabling the personal care treatment; and
- if the personal care device is determined to be in the enabled treatment condition at said first moment of time, determine treatment data relating to the personal care treatment based on the first sensor data relating to the treatment parameter at said first moment of time;
**characterized in that** the processor (217) is further configured to:
- compare the second sensor data (SD₂) with first predetermined sensor data (PSD₁) characteristic of a disabled condition of the second sensor (229) wherein the second sensor is disabled from measuring the treatment condition of the personal care device (205) with respect to the body part (203);
- determine, based on said comparing, whether the second sensor is in the disabled condition at said first moment of time (ti); and
- if the second sensor is determined to be in the disabled condition at said first moment of time, determine, based on a predefined data usage rule, whether to base the treatment data on the first sensor data (SD₁) relating to the treatment parameter at said first moment of time.

10. The personal care device as claimed in claim 8 or the personal care system (201) as claimed in claim 9, wherein:
- the enabled treatment condition of the personal care device (205) involves a condition of the personal care device in contact with the body part (203); and
- the second sensor (229) comprises a proximity sensor arranged in the personal care device such that, in the enabled treatment condition, the proximity sensor is at a predefined distance (D) from a surface (213) of the body part, wherein the proximity sensor is configured to detect the presence of said surface at said predefined distance from the proximity sensor.

11. The personal care device (1) as claimed in claim 8 or the personal care system as claimed in claim 9, wherein:
- the personal care device comprises a main housing (5) configured to be handheld by a user and a personal care treatment unit (7) coupled to the main housing by means of a coupling structure (9);
- the enabled treatment condition of the personal care device involves a condition of the personal care treatment unit in contact with the body part (3);
- the second sensor (19) comprises a pressure or force sensor (25, 27) configured to detect a pressure or force with which the user presses the personal care treatment unit against the body part; and
- the pressure or force sensor is integrated in the coupling structure.

12. The personal care device (1) as claimed in claim 8, 10 or 11, or the personal care system (201) as claimed in any of claims 9-11, wherein the first sensor (13, 215) comprises one or more of: a position sensor, a motion or displacement sensor, a velocity sensor, an acceleration sensor, an orientation sensor, a gyroscope, a magnetometer, an inertial measurement unit (IMU), a force or pressure sensor, a temperature sensor, and a timer.

13. The personal care device (1) as claimed in any of claims 8 or 10-12, or the personal care system as claimed in any of claims 9-12, wherein the processor (15) is further configured to:
- receive a plurality of the first sensor data (SD₁) and a plurality of the second sensor data (SD₂) relating to, respectively, the treatment parameter and the treatment condition of the personal care device at a plurality of successive moments of time during a treatment session;
- determine for each moment of time of said plurality of successive moments of time, based on said comparing, whether the second sensor (19) is in the disabled condition; and
- after receiving said plurality of the first sensor data and said plurality of the second sensor data, determine a portion (P) of said plurality of successive moments of time for which the second sensor is determined to be in the disabled condition;
and wherein the predefined data usage rule causes the processor to base the treatment data on:
- the first sensor data received for each moment of time of said plurality of successive moments of time if said determined portion is above a predefined threshold (P_{TH}); and
- only the first sensor data received for the moments of time of said plurality of successive moments of time for which the second sensor is determined not to be in the disabled condition if said determined portion is below said predefined threshold.

14. The personal care device as claimed in any of claims 8 or 10-13, or the personal care system (201) as claimed in any of claims 9-13, wherein the processor (217) is further configured to:
- compare the second sensor data (SD₂) with second and third predetermined sensor data (PSD₂, PSD₃) characteristic of an enabled condition of the second sensor (229) wherein the second sensor is enabled to measure the treatment condition of the personal care device (205) with respect to the body part (203), wherein the second predetermined sensor data (PSD₂) are characteristic of the enabled treatment condition of the personal care device, and wherein the third predetermined sensor data (PSD₃) are different from the first predetermined sensor data (PSD₁) and characteristic of a disabled treatment condition of the personal care device with respect to the body part wherein the treatment is not enabled;
- determine, based on said comparing of the second sensor data with said second and third predetermined sensor data, whether the personal care device is in the disabled treatment condition at said first moment of time (ti); and
- if the personal care device is determined to be in the disabled treatment condition at said first moment of time, disregard the first sensor data (SD₁) relating to the treatment parameter at said first moment of time when determining the treatment data;
and wherein the predefined data usage rule causes the processor to base the treatment data on the first sensor data relating to the treatment parameter at said first moment of time if the second sensor is determined to be in the disabled condition at said first moment of time.

15. The personal care device as claimed in any of claims 8 or 10-14, or the personal care system (201) as claimed in any of claims 9-14, further comprising a feedback unit (225, 227), wherein the processor (217) is further configured to provide, if the second sensor (229) is determined to be in the disabled condition, a feedback instruction (FB₂) to the feedback unit corresponding to feedback relating to the disabled condition of the second sensor.

16. The personal care device (1) as claimed in any of claims 8 or 10-14, or the personal care system (201) as claimed in any of claims 9-14, further comprising a feedback unit (17, 225, 227), wherein the processor (15, 217) is further configured to provide a feedback instruction (FB₁) to the feedback unit corresponding to feedback relating to the determined treatment data.

17. The personal care device (1) as claimed in any of claims 8 or 10-16, or the personal care system (201) as claimed in any of claims 9-16, wherein the processor (15, 217) is further configured to adjust an operational parameter of the personal care device (1, 205) based on the determined treatment data.
